# EUROPEAN PATENT APPLICATION

(11) **EP 3 854 451 A1**
(43) Date of publication of application: **28.07.2021**
(21) Application number: 21150618.3
(22) Date of filing: 07.01.2021
(51) Int. Cl.: A61N 5/02, A61N 5/04, A61B 18/18

(54) **CONFORMAL WEARABLE PHASED ARRAY ANTENNA SYSTEM AND METHOD**

(30) Priority: 23.01.2020 US 202016750299
(71) Applicant: Palo Alto Research Center Incorporated, Webster, NY 14580 (US)
(72) Inventor: LADAN, Shabnam, Menlo Park, CA California 94025 (US); SCHWARTZ, David Eric, Concord, MA Massachusetts 01742 (US)
(74) Representative: Gill Jennings & Every LLP

(57) **Abstract**

An apparatus comprises a flexible substrate, a phased array antenna system disposed on the substrate, and a wearable applicator. The flexible substrate is configured to conform to a shape corresponding to a portion of a human body. The phased array antenna system comprises a plurality of antenna elements configured to resonate in response to an input signal and to generate a collective output electromagnetic pattern to propagate wirelessly to a target region in a human body. The wearable applicator is configured to align the collective output electromagnetic pattern with the target region.

## Description

### TECHNICAL FIELD

This disclosure relates generally to phased array antenna systems for delivering noninvasive microwave hyperthermia treatment and methods of making the same.

### BACKGROUND

There are many different types of cancer treatment available. The treatments utilized depend on the type, extent, and location of a patient's cancer. In many circumstances more than one type of treatment is used in combination. In microwave hyperthermia, one type of cancer treatment, energy of a specific frequency, normally 915 MHz or 2.45 GHz, is delivered to a tumor using an antenna (applicator) that can be installed outside (i.e., noninvasive) or inside (i.e., invasive) a patient's body, depending on the clinical protocol. Microwave hyperthermia has been demonstrated to be an effective complementary treatment of cancerous tumors, and it is growing in use for activated targeted drug delivery.

Adding hyperthermia as an adjuvant to radiation and/or chemotherapy of tumors has been demonstrated as beneficial in clinical trials, including for superficial tumors and breast cancer recurrence in the chest wall. Studies confirm that breast cancer tumors, in particular, respond very well to microwave hyperthermia as the electrical conductivity of malignant breast tissue can be up to ten times higher than the conductivity of normal breast tissue. Data from literature, including randomized trials, have shown that combining hyperthermia with radiation improves the complete response rate and clinical response. Hyperthermia elevates tumor temperatures to a peak value between 40-43 °C for an extended period of time, such as between thirty and sixty minutes. Noninvasive microwave hyperthermia uses an applicator (e.g., an antenna device) located external to the body and close to the target. The applicator exposes the tumor to non-ionizing radiation that is absorbed in tissue leading to heating. However, the applicator should focus the radiation on the tumor while avoiding/minimizing the amount of healthy tissue exposed to the radiation.

### SUMMARY

Embodiments described herein are directed to an apparatus. The apparatus comprises a flexible substrate configured to conform to a shape corresponding to a portion of a human body. A phased array antenna system is disposed on the substrate. The phased array antenna system comprises a plurality of antenna elements configured to resonate in response to an input signal and to generate a collective output electromagnetic pattern to propagate wirelessly to a target region in a human body. A wearable applicator is configured to align the collective output electromagnetic pattern with the target region.

Other embodiments are directed to an apparatus. The apparatus comprises a flexible substrate comprising a concave surface configured to conform to a shape of a portion of a human breast. A phased array antenna system is disposed on the concave surface. The phased array antenna system comprises a plurality of antenna elements configured to resonate in response to an input signal and to generate a collective output electromagnetic pattern to propagate wirelessly to a target region in a breast. The apparatus further comprises a wearable applicator configured to align the collective output electromagnetic pattern with the target region.

Further embodiments are directed to a method. The method includes determining a location of a target region within a human body. A phased array antenna system is designed comprising a plurality of antenna elements positioned to generate a collective output electromagnetic pattern to propagate wirelessly to the target region. The phased array antenna system is disposed on a flexible substrate, and the flexible substrate is coupled to a wearable applicator. The wearable applicator is configured to align the collective output electromagnetic pattern of the phased array antenna system with the target region.

The above summary is not intended to describe each disclosed embodiment or every implementation of the present disclosure. The figures and the detailed description below more particularly exemplify illustrative embodiments.

### BRIEF DESCRIPTION OF THE DRAWINGS

The discussion below refers to the following figures, wherein the same reference number may be used to identify the similar/same component in multiple figures. However, the use of a number to refer to a component in a given figure is not intended to limit the component in another figure labeled with the same number. The figures are not necessarily to scale.
FIGS. 1-2 are schematic diagrams of a phased array antenna system in accordance with certain embodiments;
FIGS. 3-5 are diagrams of collective output electromagnetic patterns for various phased antenna array arrangements in accordance with certain embodiments;
FIG. 6 is a diagram of a phased array antenna system coupled with a wearable applicator in accordance with certain embodiments;
FIGS. 7-10 are diagrams of various phased array arrangements positioned with a wearable applicator in accordance with certain embodiments;
FIG. 11 is a schematic diagram of a collective output electromagnetic pattern directed by a phased array antenna arrangement toward a target region in accordance with certain embodiments; and
FIG. 12 is a flow diagram of a method in accordance with certain embodiments.

### DETAILED DESCRIPTION

The efficacy of hyperthermia treatment depends on the physiological properties of the tumor (e.g., severity, size, electrical properties of cancerous tissue, location, etc.) and the ability of the antenna to focus the energy (e.g., microwave radiation) on the target region (i.e., tumor). Although hyperthermia shows great promise as a complementary treatment for breast cancer, challenges limit the use of a microwave system in clinical medicine. For example, the microwave power needs to be focused locally within the heterogeneous environment of the breast.

Existing noninvasive microwave hyperthermia devices involve microwave waveguides and horn antennas that are bulky and unable to conform to the human body. For example, they form a structural enclosure that a patient enters, or lies within, that encircles at least the portion of the patient's body containing the tumor(s). A single device is designed to accommodate a variety of different-sized patients and/or body portions. These systems incorporate rigid antennas, are highly complex, and require a patient to receive hyperthermia treatment at a hospital or clinic. The patient must lie down for a prolonged period of time with a bulky and heavy applicator compressed around the portion of the body containing the tumor(s). For example, a breast cancer patient is required to place her/his breast into the cavity of a large machine and endure uncomfortable physical pressure and heat stress for thirty to sixty minutes. Such devices also produce a heating pattern that is not adjustable for a specific patient's anatomy and does not accommodate variable tissue types and blood perfusion.

Efforts to improve efficacy of hyperthermia treatment may include placing the applicator closer to the patient to reduce the amount of energy used and to better focus on the tumor. They may also include having a treatment device that is adaptable for specific tumor locations, tumor and/or tissue properties, and patient anatomy. Further, improving comfort when using a device would help to increase compliance with the treatment. Embodiments described herein are directed to noninvasive microwave hyperthermia treatment devices utilizing a compact design of a phased array antenna system on a flexible substrate. The flexible substrate is coupled with a wearable applicator designed to have a small form factor to align the radiation output of the antenna array to a target region (e.g., tumor) in a patient's body. In certain embodiments, the wearable applicator is conformable and designed as a brassiere, or an insert for a cup of a brassiere, to treat breast tumors. For example, the hyperthermia treatment devices described herein may be used in combination with radiation or chemotherapy to accelerate the treatment strategy in both stage one and stage two superficial breast tumors. In further embodiments, the treatment devices may be used as vehicles for, or in conjunction with, drug delivery.

Embodiments described herein combine a small form factor with the ability to locally focus energy at a target region from a location in close proximity to the target region. For example, a bra-shaped system of compact, wearable, conformal phased array antennas is provided for use in noninvasive microwave treatment for breast tumors. Flexible electronics allow disposition of a phased array antenna system on a flexible substrate to create a conformal, compact, and lightweight device. This device is then integrated into a wearable applicator such as a brassiere, which increases a patient's comfort during treatment as well as placing the device closer to the treatment area. The ability to wear a coherent phased array device close to a patient's body provides local focusing where the transmitted microwave signals are constructively combined at the targeted tumor location and cancel each other out elsewhere to minimize damage to healthy tissues near and surrounding the tumor. Operation of the phased array antenna system is further discussed below.

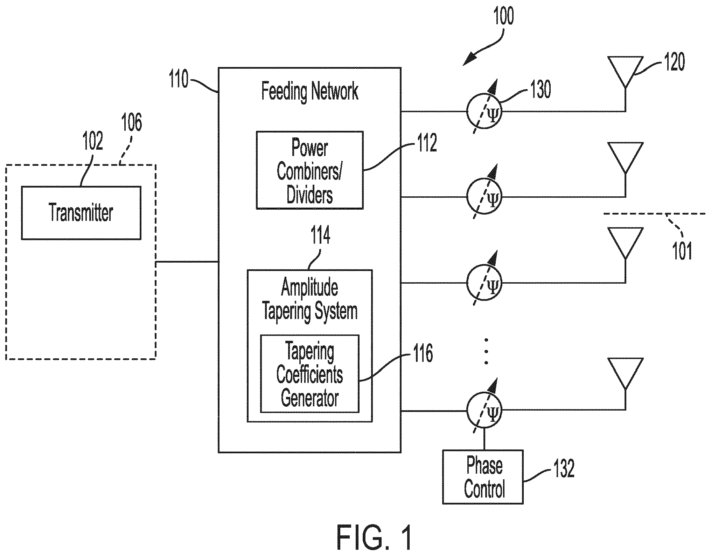

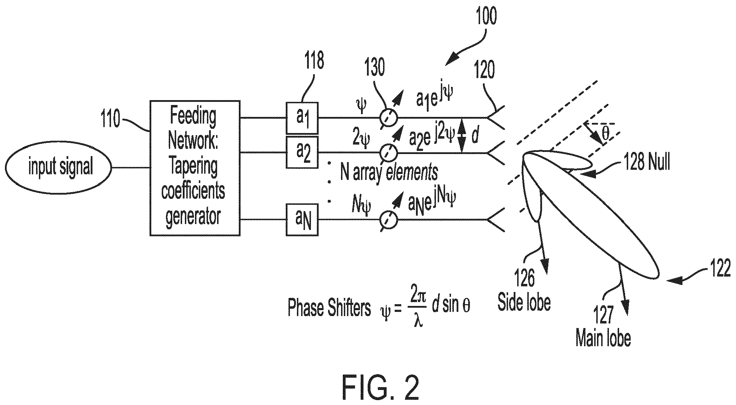

FIGS. 1 and 2 illustrate a phased array antenna system 100 configured to couple to a radio frequency (RF) transmitter 102. In some configurations, the phased array antenna system 100 is configured to couple to an RF transceiver 106, which includes both the RF transmitter 102 and a RF receiver. The RF transmitter 102 is coupled to a feeding network 110. The phased array antenna system 100 includes a plurality of antennas 120. According to some embodiments, each of the antennas 120 is coupled to one of a plurality of phase shifters 130 and to the feeding network 110. The plurality of phase shifters 130 may be designed for passive or active beam steering, which is illustrated by a combination of a circle (representing passive beam steering) and an arrow having a dashed line (representing an optional, active phase shifter). A phase control 132, which can include one or more processors among other components, is operably coupled to the phase shifters 130, e.g., in embodiments involving active beam steering. In certain embodiments involving active beam steering, the phase control 132 is configured to adjust a phase shift of each of the phase shifters 130 to electronically steer an antenna array pattern 122, such as in one or both of an azimuth plane and an elevation plane. The phase shifters 130 rotate the antenna array pattern 122 after an RF signal is coupled to the antennas 120 through the feeding network 110. The antenna array pattern 122 can be steered by the phase control 132 and the phase shifters 130 when the phased array antenna system 100 operates in a transmit mode.

The antennas 120 of the phased array antenna system 100 cooperate to create a beam of radio waves, e.g., microwaves, that can be fixed at, or electronically steered to, a location in a desired direction without moving the antennas 120. The antennas 120 can also be electronically steered to a location in a desired direction when receiving radio waves from a target source or to avoid external sources of interference. In a transmit mode, radio frequency current generated by the RF transmitter 102 is fed to the feeding network 110 and to the individual antennas 120 with the correct phase relationship via the phase shifters 130 so that the radio waves from the separate antennas 120 add together to increase the radiation in a desired direction, while canceling or suppressing radiation in undesired directions. By changing the phase of the phase shifters 130, the phase control 132 can change the angle or angles of the main beam 127 and null(s) 128 of the antenna array pattern 122. For example, the phase control 132 can adjust the phase of the phase shifters 130 to cause the antenna array pattern 122 to be directed at a desired angle (e.g., an azimuth angle or an elevation angle) or angles relative to an axis 101 of the phased array antenna system 100.

According to certain embodiments, the plurality of antennas 120 is designed to generate a beam of radio waves, e.g., microwaves, directed in a fixed beam direction. The fixed direction of the antenna array pattern 122 can be any desired direction. The beam forming is done passively, as opposed to the above-discussed active beam forming, through the number of antenna elements, their arrangements with respect to each other, and the relative phase shifting between each antenna element. In certain embodiments, a fixed pattern is formed using phase shifters 130 that are not controllable, e.g., delay lines. Passive beam forming is achieved through designing an array prior to application, or use, of the antenna system 100. According to embodiments that do not include the phase shifters 130, or phase control 132, each of the antennas 120 is coupled to the feeding network 110.

The feeding network 110 includes power combiners/dividers 112. Depending on the particular configuration of the phased array antenna system 100, the feeding network 110 can include a single power combiner/divider 112 or, in more complex configurations, any number of power combiners/dividers 112. The power combiners/dividers 112 can be implemented as a Wilkinson power divider, a hybrid coupler, a directional coupler, or any other circuit that can combine and/or divide signals. Each of the power combiners/dividers 112 can combine and/or divide signals passing through the feeding network 110. For example, the power combiners/dividers 112 can be configured to split a common RF signal generated by the transmitter 102 between a multiplicity of antennas 120.

The feeding network 110 may also include an amplitude tapering system 114. Among other components, the amplitude tapering system 114 includes a generator 116 configured to generate tapering (weighting) coefficients 118. The amplitude tapering system 114 is configured to generate amplitude tapering coefficients 118 via the tapering coefficients generator 116 and apply an amplitude tapering function on a transmitted radio frequency signal. The amplitude tapering function applied by the amplitude tapering system 114 can comprise a combination of two or more disparate amplitude tapering functions.

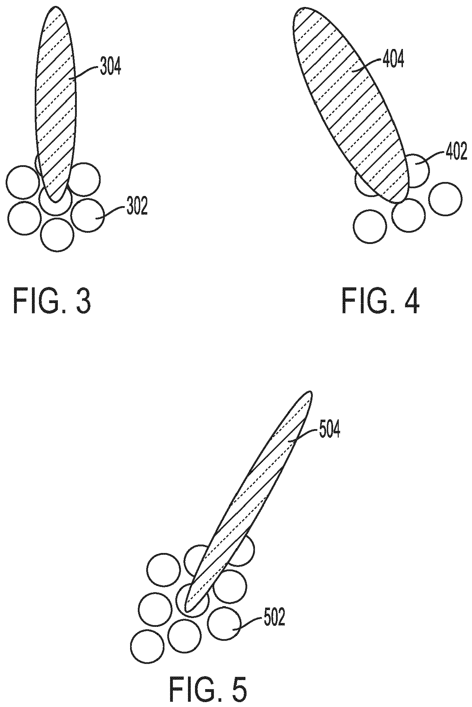

The collective output of a phased array antenna system varies depending on the configuration of the antennas in the array and/or the electronic control of the system, when using an active beam steering network. FIGS. 3-5 illustrate various output beams for different antenna configurations. In each of the figures, only the main lobe is shown for ease of illustration, but it is understood that one or more side lobes may also be present. FIG. 3 shows a circular antenna array pattern 302 that produces a collective output of electromagnetic radiation 304 (e.g., a beam). The beam is directed approximately perpendicular to the array. However, the shape, size and direction of the beam may be altered by changing the antenna array design. For example, FIG. 4 shows a triangular antenna array pattern 402 that produces a collective beam 404 directed at an angle and having a thickness greater than that of beam 304. Alternatively, FIG. 5 shows a diamond-like antenna array pattern 502 that produces a collective beam 504 directed at an angle different from that of beam 404 and having a thickness less than that of beam 304. In addition to varying the antenna array pattern/design, the size, shape, and direction of the collective beam is passively controlled by the phase delays to each antenna element when using a fixed phase antenna system. The main beam is generated by controlling the number of antenna elements, the arrangement of the elements, and the relative phase shifting between each element. In further embodiments, the beam forming may be actively controlled through active phase shifters and amplitude control as discussed above.

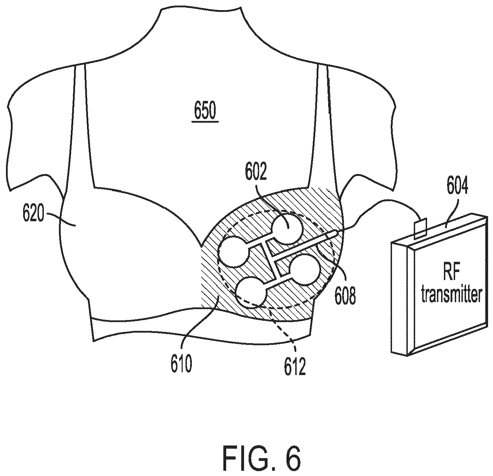

The phased array antenna system can be disposed on a flexible substrate 612, which allows the system to be formed into a wearable applicator, as shown in FIG. 6. In the figure, a single antenna 602 is shown as part of an array of antennas. The array includes connecting circuitry and a power feed 608 coupled to an energy source 604 such as a radio frequency transmitter. The energy source 604 provides an input signal to the array and is preferably portable, but it can also be a stationary device. The antennas and connecting circuitry may be disposed on, or embedded in, a flexible substrate 612 as is known. While not shown, the system includes additional components such as resistors, capacitors, phase shifter integrated circuits, varactor diodes, etc. that may be non-printed components coupled to the flexible substrate 612 through other means such as direct die attach techniques. In certain embodiments, however, the antenna array may be printed on a polymeric substrate. The flexible substrate 612 is coupled to a wearable applicator 610. The wearable applicator 610 is configured to align the collective output of the phased array antenna system with a target region (e.g., tumor) within the body 650. The wearable applicator may be an insert for an existing wearable device, or the wearable applicator may be a garment. For example, wearable device 610 is shown as part of a brassiere 620 configured to treat tumors located within a breast. Here, the wearable applicator 610 may either be an insert into an existing cup of brassiere 620 or form a part of, or the whole, cup of brassiere 620.

In addition, the hyperthermia treatment device may include a cooling pad disposed between the antenna elements of the phased array and the target region, e.g., against a patient's skin. The cooling pad may have the same areal size and shape as the wearable applicator, or it may be smaller or larger. In certain embodiments, the cooling pad may only be disposed over the antenna elements of the phased array. The cooling pad is designed with a thickness and coverage area to minimize interference with the formation and shaping of the collective output beam. In certain embodiments, the cooling pad is disposed as an inner layer including a cooling gel, such as silicon oil or a hydrogel, to maintain a comfortable skin temperature during the hyperthermia treatment.

As may be seen, the conformal bra-shaped wearable applicator positions the phased array antenna system external but in close proximity to the tumor location within the body 650. This provides for delivery of increased, or maximum, energy with decreased, or minimal, attenuation while being a lightweight, wearable, and ergonomic treatment device. The wearable nature of the applicator, especially when in the form of a brassiere or brassiere insert, allows the treatment device to move with a patient's body, including consistently with a patient's breathing. This enables consistent and effective energy delivery as well as increased comfort for the patient. It also makes the system portable, when used with a portable energy source, which can allow for treatment to occur in a wide variety of clinical settings, including at-home treatment.

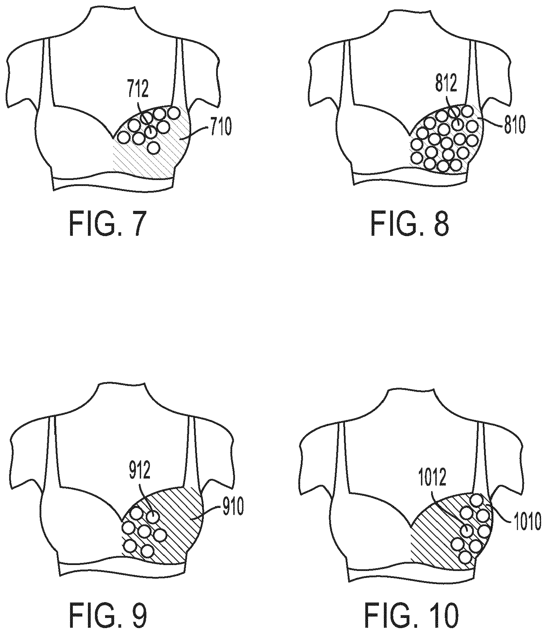

The hyperthermia treatment devices described herein are customizable for each specific patient. By positioning the array of antenna elements proximate a patient's skin, the heating depth, location, phase delays, and number of antenna elements in the array are designed based on the anatomy of the patient and the type of tumor. This may be seen in FIGS. 7-10, which illustrate various configurations and locations of the antenna elements in the array. For example, FIG. 7 illustrates an array 712 positioned on a wearable applicator 710 across the top of a breast that may form a collective output pattern at an angle to reach a target region in an upper/central portion of the breast and/or chest cavity. FIG. 8 illustrates an array 812 on a wearable applicator 810 with coverage over about the entire breast. Such an array may be used, for example, when multiple tumors are present. Alternatively, the design of FIG. 8 may be utilized as a mass production article where customization of the collective output radiation pattern is controlled electronically through beam steering and amplitude adjustment. FIG. 9 illustrates an array 912 on a wearable applicator 910 configured to form an output pattern directed toward a target region more centrally located in the chest cavity. In certain embodiments, array 912 may produce a radiation pattern similar to that of FIG. 3. FIG. 10 illustrates an array 1012 positioned on a wearable applicator 1010 proximate the side of the breast. The collective output pattern of array 1012 may be directed to reach a target region in, or near, a patient's armpit or along the side of the chest cavity. While FIGS. 7-10 illustrate a wearable applicator inserted in, or integrated into, only one cup of a brassiere, the wearable applicator may be shaped to cover all, or portions of, one or both cups and/or the band and/or the straps of a brassiere. Also, while the wearable applicators are shown as coinciding with an entire brassiere cup, they could be any variety of shapes and sizes that conform to all, or a portion, of a patient's body (e.g., a breast).

The hyperthermia treatment devices described herein may be further customized through passive or electronic control of the collective output radiation pattern and power profile. For example, the phased array antenna system includes an adjustable power profile to accommodate variable tissue types and blood perfusion. Control of the output radiation pattern allows for improved focus on a target region (e.g., a tumor or disease location).

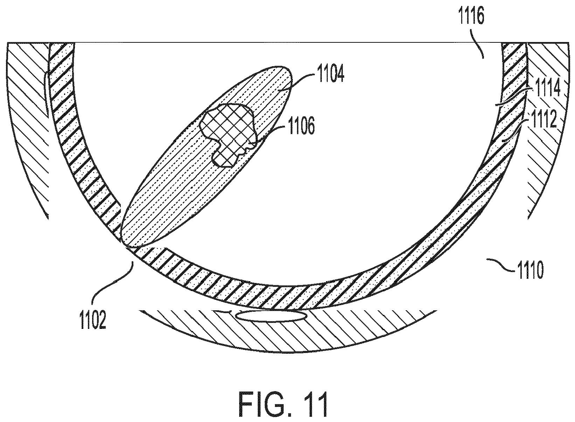

FIG. 11 illustrates a collective output electromagnetic radiation pattern focused on a tumor target region. The target region 1106 is located within a cancer patient's breast tissue 1116. The hyperthermia treatment device includes a cooling pad 1112 proximate the patient's skin 1114. Antenna elements of a phased array system are disposed on, or embedded in, the wearable applicator 1110. As discussed above, the array of antenna elements is set to form a predetermined collective output radiation pattern 1104 designed to reach and focus on the target region.

To accomplish the customized focus on a specific patient's tumor, the positioning of the antenna elements on the wearable applicator and/or the wearable applicator itself is designed to align the output radiation pattern with the patient's tumor location. For example, the size and shape of the applicator may be predetermined and conformed to a patient's breast such that disposition of the antenna elements on the wearable applicator will provide the necessary alignment. In certain embodiments, the antenna array is designed and coupled to an applicator having integrated alignment elements specific to a patient's anatomy. For example, such a wearable applicator would be inserted at a specific position within a brassiere cup and/or adhered to a patient's body at a designated position. Thus, the number of antenna elements, the antenna array design, the relative phase delays, the collective output electromagnetic pattern, the input power, and the wearable applicator size/shape/position/type are all customizable to provide targeted focus of the treatment energy on the tumor target region and minimize/avoid exposure to healthy tissue.

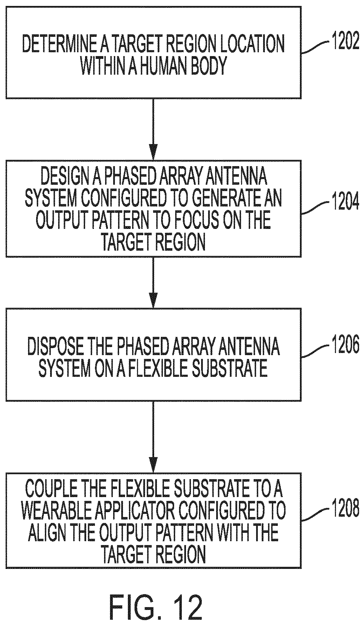

A method for fabricating the hyperthermia devices described herein is illustrated in the flow chart of FIG. 12. Since the device is customized to a patient's anatomy and tumor, a target region (e.g., tumor) location is determined within the patient's body 1202. This may be done with known medical imaging and measuring techniques. Based on the size and location of the target region (including the depth within the body), a phased array antenna system is designed 1204. The design includes the number of antenna elements as well as the pattern for placement in relation to each other. The antenna design may include single and/or multi-layer resonant structures. The array design accommodates the ultimate shape of the treatment device, such as taking into account a concave surface conformed to a patient's anatomy (e.g., a breast). The array is designed to generate a collective output electromagnetic pattern that reaches, and focuses on, the target region. The shape and size of the output pattern is also designed to have minimal exposure to healthy tissue surrounding the target region. The phased array antenna system is disposed on, or embedded in, a flexible substrate 1206. For example, the antenna elements, and corresponding circuitry, may be printed on a polymer substrate. The flexible substrate is coupled to a wearable applicator and the combination is configured to align the generated output pattern with the target region 1208.

To focus the generated output beam on the targeted region, the antenna elements may be positioned on the flexible substrate such that a patient may fasten the wearable applicator to their body and the antenna elements will have a predetermined alignment with the target region. For example, the wearable applicator may have a predetermined shape and the flexible substrate may have a corresponding shape so that the disposition of the antenna elements on the flexible substrate provides the requisite alignment when the wearable applicator is worn. Alternatively, the flexible substrate may have a different size/shape than the wearable applicator such that coupling the flexible substrate to the wearable applicator provides the necessary alignment. For example, this may occur when the wearable applicator is a full garment such as a brassiere. In certain embodiments, the wearable applicator may include adhesive and/or alignment features (e.g., lines, notches, coupling elements, etc.) to position the applicator directly on the body.

As set forth above, various embodiments directed to hyperthermia treatment devices include a phased array antenna system coupled to a wearable applicator to conform to a patient's body. The proximity and wearable nature of the treatment device provides targeted and locally focused heating of tumor tissue material by leveraging advances in conformal phased array antenna systems. Further, the ability to customize the treatment devices to each patient's anatomy and tumor type improves the efficacy of, and likelihood of compliance with, prescribed treatment.

Unless otherwise indicated, all numbers expressing feature sizes, amounts, and physical properties used in the specification and claims are to be understood as being modified in all instances by the term "about." Accordingly, unless indicated to the contrary, the numerical parameters set forth in the foregoing specification and attached claims are approximations that can vary depending upon the desired properties sought to be obtained by those skilled in the art utilizing the teachings disclosed herein. The use of numerical ranges by endpoints includes all numbers within that range (e.g. 1 to 5 includes 1, 1.5, 2, 2.75, 3, 3.80, 4, and 5) and any range within that range.

The foregoing description has been presented for the purposes of illustration and description. It is not intended to be exhaustive or to limit the embodiments to the precise form disclosed. Many modifications and variations are possible in light of the above teachings. Any or all features of the disclosed embodiments can be applied individually or in any combination and are not meant to be limiting, but purely illustrative. It is intended that the scope of the invention be limited not with this detailed description, but rather, determined by the claims appended hereto.

## Claims

1. An apparatus comprising:
a flexible substrate configured to conform to a shape corresponding to a portion of a human body;
a phased array antenna system disposed on the substrate, the phased array antenna system comprising a plurality of antenna elements configured to resonate in response to an input signal and to generate a collective output electromagnetic pattern to propagate wirelessly to a target region in a human body; and
a wearable applicator configured to align the collective output electromagnetic pattern with the target region.

2. The apparatus of claim 1, wherein the wearable applicator is configured to secure the apparatus to the portion of the human body.

3. The apparatus of claim 1, wherein the phased array antenna system is coupled to a controller configured to adjust input power transmitted to one or more of the antenna elements.

4. The apparatus of claim 1, wherein the phased array antenna system is coupled to a controller configured to dynamically shape and direct the collective output electromagnetic pattern.

5. The apparatus of claim 1, wherein an RF signal from a dedicated power source is coupled to the phased array antenna system.

6. The apparatus of claim 1, wherein at least one of the apparatus size, apparatus shape, output electromagnetic pattern, input power, and wearable applicator is designed for a specific patient.

7. The apparatus of claim 1, wherein the target region is within a breast.

8. The apparatus of claim 1, wherein the target region is a tumor.

9. The apparatus of claim 1, further comprising a cooling pad disposed between the plurality of antenna elements and the target region.

10. An apparatus comprising:
a flexible substrate comprising a concave surface configured to conform to a shape of a portion of a human breast;
a phased array antenna system disposed on the concave surface, the phased array antenna system comprising a plurality of antenna elements configured to resonate in response to an input signal and to generate a collective output electromagnetic pattern to propagate wirelessly to a target region in a breast; and
a wearable applicator configured to align the collective output electromagnetic pattern with the target region.

11. The apparatus of claim 10, wherein the wearable applicator is an insert configured to secure the apparatus within the cup of a brassiere.

12. The apparatus of claim 10, wherein the wearable applicator is a brassiere.

13. The apparatus of claim 10, wherein the phased array antenna system is coupled to a controller configured to adjust power transmitted to one or more of the antenna elements.

14. The apparatus of claim 10, wherein the plurality of antenna elements are single layer resonant structures.

15. The apparatus of claim 10, wherein the plurality of antenna elements are multi-layer resonant structures.

16. The apparatus of claim 10, further comprising a cooling pad disposed between the plurality of antenna elements and the target region.

17. The apparatus of claim 10, wherein the phased array antenna system moves consistently with a person's breathing.

18. The apparatus of claim 10, wherein the collective output electromagnetic pattern generates a temperature of between about 40°C and 43°C at the targeted region over a period of time.

19. A method comprising:
determining a location of a target region within a human body;
designing a phased array antenna system comprising a plurality of antenna elements positioned to generate a collective output electromagnetic pattern to propagate wirelessly to the target region;
disposing the phased array antenna system on a flexible substrate; and
coupling the flexible substrate to a wearable applicator configured to align the collective output electromagnetic pattern of the phased array antenna system with the target region.

20. The method of claim 19, further comprising coupling a cooling pad to a surface of the flexible substrate.
